# FASCICULE DE BREVET EUROPEEN

(11) **EP 3 285 822 B1**
(45) Date de publication et mention de la délivrance du brevet: **19.06.2019**
(21) Numéro de dépôt: 15831059.9
(22) Date de dépôt: 28.12.2015
(51) Int. Cl.: A61M 1/10

(54) **DISPOSITIF DE COEUR ARTIFICIEL INTÉGRAL**
INTEGRIERTE KÜNSTLICHE HERZVORRICHTUNG
INTEGRAL ARTIFICIAL HEART DEVICE

(30) Priorité: 05.01.2015 FR 1500004; 24.12.2015 FR 1502697
(43) Date de publication de la demande: 28.02.2018
(73) Titulaire: Plas, Gérard, 34190 Brissac (FR); Plas, Corentin, 34190 Brissac (FR); Plas, Gwen Sébastien, Penn Valley, CA 95946 (US)
(72) Inventeur: Plas, Gérard, 34190 Brissac (FR); Plas, Corentin, 34190 Brissac (FR); Plas, Gwen Sébastien, Penn Valley, CA 95946 (US)
(74) Mandataire: Rhein, Alain
(86) Numéro de dépôt international: PCT/FR2015/000243
(87) Numéro de publication internationale: WO 2016/110613

(56) Documents cités:
- US-A- 3 771 173
- US-A- 3 919 722
- US-A- 4 152 785

## Description

Le dispositif revendiqué, dont il est question ci-après, est un dispositif de coeur artificiel intégral destiné à être implanté dans la cavité péricardique en remplacement du coeur biologique.

Il existe un certain nombre de dispositifs d'appoint, partiels et d'utilisation temporaire, en général en assistance du ventricule gauche, qui préludent à l'ambition de disposer d'un coeur artificiel de remplacement global qualifié, par les propositions actuelles, de coeur artificiel intégral.

Abstraction faite des dispositifs déposés qui n'ont pas été mis en oeuvre, et des tentatives de mise en oeuvre de pompes rotatives, inadaptées, tous les coeurs proposés ayant été expérimentés sont caractérisés par le fait que les battements ventriculaires sont alternatifs et successifs, et non simultanés. La raison semble en être que ces propositions ne comportent pas d'atrium dynamique (également nommés oreillettes selon les terminologies, étant entendu que nous faisons élection du terme atrium pour le reste de la rédaction), de telle sorte que, dans ce cas de figure particulier, la durée de la diastole est obligatoirement égale à la durée du battement, à savoir, une durée qui, dans le fonctionnement naturel, simultané, est égale au total de la durée de la systole additionnée de la durée distincte de la diastole telle que ce terme s'entend, c'est-à-dire la durée du remplissage des corps de pompes ventriculaires et non la durée du retour permanent du volume systolique initial qui correspond à la durée du battement, comme il faut l'entendre si on veut considérer l'intervention des atriums et leurs fonctionnements comme partie intégrante du système retour veineux du circuit fermé sanguin, étant entendu que cette section du circuit veineux se trouve à l'intérieur de l'appareil cardiaque biologique.

Pour contourner la contradiction qui découle de ce qui précède, les promoteurs jusqu'à ce jour ont été contraints d'additionner les deux durées séquentielles constitutives de la durée du battement en substitution de la durée de la diastole de telle sorte que chaque demi-coeur à besoin de la durée totale du battement pour remplir son ventricule, ce qui implique l'alternativité des battements.

L'état de la technique démontre que, si la création des pompes ventriculaires ne pose aucun problème réel, en revanche, la création d'atriums efficaces n'a encore jamais reçu de solution raisonnable. Le coeur biologique est un appareil qui est capable de faire la synthèse harmonieuse de deux systèmes circulatoires consécutifs, certes, mais fondamentalement différents, contradictoires et incompatibles sans le concours d'un dispositif de coordination et d'harmonisation, intermédiaire et synthétique, dans le processus d'accueil du sang veineux de retour durant la systole, par le moyen d'un stockage temporaire et consécutivement du remplissage des ventricules, en deux phases, durant la diastole.

En effet la systole, ou phase de saccade de la chasse du sang vers les organes, est séquentielle et spasmodique dans le réseau artériel, à contrario de la diastole ou phase de remplissage des ventricules, qui s'opère elle-aussi de façon séquentielle, du moins en apparence, mais à partir d'un flux retour veineux qui lui, par contre, est permanent, constant et régulier, lequel phénomène est incompatible avec le fractionnement du temps de battement global divisé en systole et diastole, compte tenue de l'acception communément admise du terme « diastole ». En effet, si l'accès des ventricules est interdit au flux sanguin, veineux, de retour, durant la systole, l'accès du coeur, de fait, serait interdit au flux retour du circuit fermé sans l'intervention des atriums, adaptables aux conditions du moment, variables en permanence, et que sans l'intervention de ce dispositif de stockage temporaire, actif, réactif, adaptable et synchrone, le flux veineux permanent de retour serait interrompu de façon séquentielle provoquant des coups de bélier arrières, destructeurs dans le système veineux, sauf à admettre que le circuit sanguin veineux de retour ne s'arrête pas à l'entrée des atriums mais devant l'orifice mitral pour le demi-coeur gauche et l'orifice tricuspide pour le demi-coeur droit.
En conséquence de ce qui précède, les atriums sont incontournables.

Par conséquent, abstraction faite d'une organisation générale elle-même originale, l'essentiel de l'invention revendiquée repose dans la proposition d'un dispositif de temporisation en substitut des atriums naturels qui a fait défaut aux propositions antérieures de l'état de la technique de telle sorte que le dispositif revendiqué est un coeur artificiel de substitution totalement intégral, qui comporte deux ventricules artificiels à pulsions simultanées, séquentielles et spasmodiques, droit et gauche, assortis de leurs atriums artificiels respectifs, droit et gauche, réactifs, synchrones et adaptables, ainsi que les valves anti-retour correspondantes, nécessaires à l'organisation des sens uniques de circulation. Les ventricules et les atriums sont animés, d'un même mouvement, par un dispositif électromagnétique original, qui ne comporte aucune pièce mécanique de transmission susceptible de panne, lequel dispositif est contrôlé par un dispositif électronique de pilotage de l'électro-aimant, pour les variations de débits, en fonction des paramètres recueillis par des capteurs de gaz carbonique et d'adrénaline implantés dans le coeur droit et deux barorécepteurs implantés dans le ventricule et dans l'atriums du coeur gauche.

Les dessins annexés illustrent l'invention :
La figure 1 représente en perspective cavalière le dispositif de l'invention en fin de diastole.
La figure 2 représente en perspective cavalière le dispositif de l'invention en fin de systole.
La figure 3 représente en coupe de face le dispositif de l'invention en fin de diastole.
La figure 4 représente en coupe de face le dispositif de l'invention en fin de systole.
La figure 5 représente en coupe de côté le dispositif de l'invention en fin de diastole.
La figure 6 représente un schéma simplifié du dispositif de refroidissement de l'électro-aimant.
La figure 7 représente de façon schématique les valves circulaires, la partie supérieure représentant une valve artérielle et la partie inférieure représentant une valve veineuse.
La figure 8 représente de façon simplifiée le mode de construction des valves lamellaires.

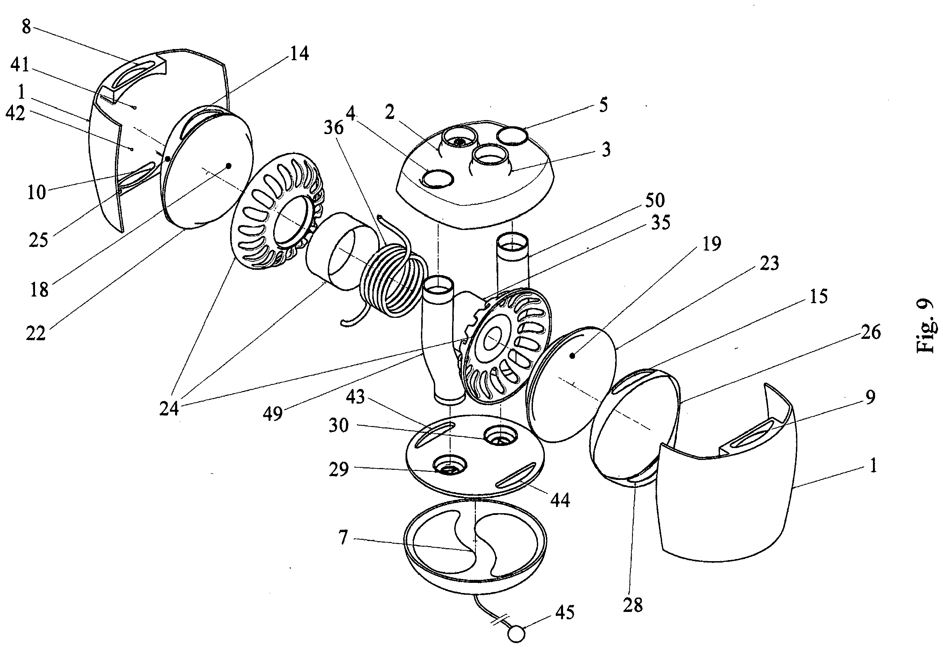

La figure 9 représente en disposition éclatée le dispositif de l'invention en fin de diastole.

En référence à ces dessins, le dispositif comporte une coque rigide (1), de type ovoïde, comprenant en extérieur des appendices indispensables de raccordement, artériels (2, 3), et veineux (4, 5), au réseau vasculaire biologique servi et qui comporte, à l'intérieur, divers éléments de cloisonnement secondaires (6, 7, 8, 9, 10, 11), mais qui font partie intégrante de la coque au même titre que les appendices de communication et de raccordement extérieurs précités.

La coque a pour double fonction, de première part, d'isoler le dispositif de coeur artificiel revendiqué du reste de l'organisme dans la cavité péricardique et de deuxième part de faire fonction de squelette pour l'ancrage de l'architecture interne des dispositifs secondaires concourants.

De ce qui précède, l'espace volumétrique intérieur de la coque (1) est organisé de façon à délimiter quatre types d'espaces volumétriques spécifiques, résultants, à savoir, A, B, C, hydrauliques et D, pneumatique, caractérisés par leurs fonctions ou par les fonctions des dispositifs secondaires concourants qui les occupent. Trois de ces types, A, B, et C sont représentés par paires, respectivement identifiés par A1 et A2, B1 et B2, C1 et C2, et le quatrième, D, est unique, ce qui porte à sept le nombre effectif des espaces volumétriques internes, tels que chacun est étanche par rapport aux six autres, abstraction faite des moyens de communication nécessaires qui les joignent, lesquelles sont contrôlées par des valves anti-retour qui assurent le sens unique des circulations conventionnelles des demi-coeurs droit et gauche selon le modèle biologique.

Les espaces volumétriques A1 et A2, hydrauliques, sont des pompes à membranes qui assurent la fonction ventriculaire et dont l'espace volumétrique variable est défini par la surface des membranes (18, 19) et leur course, solidaires d'un bâti (22, 23), de maintien et de présentation, circulaire et rigide, lequel bâti est prolongée par une jupe cylindrique (25, 26) qui assure l'étanchéité du compartiment avec la partie de la coque en vis-à-vis, qui correspond à la projection du plan circulaire des dites membranes sur la surface interne de la dite coque (1).

Les deux membranes (18, 19) sont positionnées dos-à-dos et les bâtis rigides circulaires (22, 23) qui les maintiennent dans leur plans parallèles sont solidaires entre eux par une structure squelettique (24) qui résulte d'une combinaison géométrique de deux coupelles ajourée et d'un cylindre intercalaire, qui circonvient un électro-aimant refroidit, de façon concentrique, telle que ladite structure fixe et maintient la distance qui sépare lesdites membranes ainsi que la distance qui sépare de la coque les bâtis circulaires (22, 23) des membranes (18, 19).

Les jupes cylindriques (25, 26) qui prolongent les bâtis circulaires des membranes jusqu'au contact de la coque (1) ferment l'espace volumétrique variable dans lequel le battement des membranes assure la mise en pression des sangs veineux.

Les jupes (25, 26) sont assorties de lumières oblongues (14, 15, 27, 28), à savoir deux par espace volumétrique ventriculaire, l'une pour admettre le sang veineux (27, 28) et l'autre (14, 15) qui autorise son expulsion vers les artères.

Les membranes (18, 19) hébergent en leur centre et dans leur épaisseur, des disques d'aimants permanents (20, 21), inclus dans l'élastomère des dites membranes.

Les aimants permanents inclus dans les membranes, sont installés l'un par rapport à l'autre de telle sorte que le pôle nord de l'un (20) regarde le pôle sud de l'autre (21).

La structure de liaison (24) des dispositifs ventriculaires accueille et maintient en son centre un électro-aimant refroidi, à noyau fixe (35), orienté et alimenté de telle sorte que le pôle nord de l'électro-aimant soit opposable au pôle nord de l'un des aimants permanents (20), et que le pôle sud de l'électro-aimant soit opposable au pôle sud de l'autre aimant permanent (21), ou inversement, fonction de l'alimentation de l'électro-aimant.

Le dispositif, constitué par les aimants permanents (20, 21) inclus dans les membranes ventriculaires (18, 19), la structure de liaison squelettique (24) et l'électro-aimant (35) qui anime l'ensemble, constitue le dispositif d'animation général, à double action, hydraulique pour les espaces ventriculaires (A1 et A2), et pneumatique pour l'espace volumétrique (D) décrit ci-après.

Le dispositif d'animation générale est tangent par la circonférence des bâtis (22, 23), et des coupelles de la structure de liaison, (24), à la cloison d'isolement (6) dont le plan est parallèle à l'axe passant par le centre des coupelles et du dispositif d'animation général électromagnétique, laquelle cloison (6) isole dans la coque un deuxième type d'espace volumétrique hydraulique, B, de transit et de retournement, divisé en deux, B1 et B2, par la cloison de subdivision (7) qui lui est perpendiculaire, lesquels espaces B1 et B2 accueillent et retournent les sangs veineux en provenance des atriums C1 et C2 par deux lumières circulaires (29, 30), ouvertes dans la dite cloison d'isolement (6) et les dirigent vers les lumières d'admission oblongues (27, 28) des ventricules droit (A1) et gauche (A2) grâce à des lumières oblongues (43, 44) de sorties des espaces B1 et B2, pratiquées dans la cloison d'isolement (6) en vis-à-vis des lumières oblongues (27, 28) avec lesquelles elles communiquent par des conduits (10 et 11), en double cloison rabattue, de section oblongue.

Les espaces de transit B1 et B2 sont solidaires des atriums C1 et C2, et communiquent avec ces derniers par les deux lumières oblongues (27, 28) comme il a été dit, lesquelles sont équipées de valves anti-retour à sens unique (31, 32).

Accessoirement, les jupes cylindrique (25 et 26), solidaires des bâtis, et des coupelles, comme il a été dit, sont tangentes au plan de la cloison d'isolement (6), plane, de telle sorte que de la lumière de sortie oblongue de la cloison d'isolement (6) à la lumière oblongue des jupes cylindriques (25, 26), qui leurs correspond, comme il a été dit précédemment, il est nécessaire d'enfermer les deux dites lumières dans des conduits en double cloison rabattue (10, 11), solidaire de la coque, de la cloison (6) et de la jupe, de telle sorte que ces appendices accessoires (10, 11) de la coque (1) assurent l'étanchéité des espaces communicants (B1 et A1, B2 et A2), par rapport à tous les autres espaces volumétriques et conduisent les flux veineux des espaces d'atriums (C1, C2), via les espaces de transit (B1, B2) vers les lumières (25, 26) des jupes ventriculaires.

Les espaces hydrauliques C1 et C2 sont constitués par deux tubulures élastiques (49, 50), irrégulières, identiques et symétriques, à volume variable, qui joignent les appendices de raccordement veineux de la coque (4, 5) aux lumières circulaires (29, 30) de la cloison d'isolement (6), lesquels espaces C1 et C2 sont à volume variable et préformés pour pouvoir envelopper la structure de liaison squelettique (24), sans que les dites tubulures (49, 50) entrent en contact surfacique, ni avec la coque, ni avec la structure de liaison squelettique (24) du dispositif d'animation général électromagnétique dans le cas de sa dilatation maximum, laquelle dilatation doit pouvoir accepter la moitié du volume systolique maximum additionné du volume mort constatable en phase de relaxation, qui doit être par construction le plus petit possible.

L'addition des espaces A1 et A2, B1 et B2 et C1 et C2 déterminent, par soustraction du volume total, interne, de la coque (1), un espace volumétrique unique D, qui accueille la structure (24) et l'électro-aimant (35), mais aussi les atriums immergés C1 et C2, tel que l'espace volumétrique D est occupé par un gaz, fonction de sa densité et de son coefficient d'élasticité, lequel gaz fait fonction de transmission pneumatique élastique et de ressort pneumatique diffus, de telle sorte que, par construction, en vertu du battement commun des membranes ventriculaires dans l'espace D, le dit espace D est lui aussi à volume et à dépression variable, et que la variation de la dépression pneumatique dans l'espace D agit sur les parois des tubulures (49, 50) des atriums (C1, C2) pour modifier leur résistance à la pression sanguine veineuse, interne, constante, de retour, qui s'exprime à l'intérieur des parois desdites tubulures (49, 50) des atriums C1 et C2.

C'est cette particularité, à savoir l'occupation du dit espace D par un gaz et son comportement pneumatique à dépression variable, qui fonde l'originalité principale du dispositif revendiqué de coeur artificiel, en ce que ce gaz, du fait de son élasticité, absorbe une fraction du couple dépresseur mené par les déplacements vers les extérieurs des membranes ventriculaires, de telle sorte qu'une fraction seulement du dit couple se traduit par un couple dépressionnaire effectif, actif et relatif, sur les parois des tubulures (49, 50) d'atrium C1 et C2, lesquels atriums sont de ce fait conditionnés pour ne recevoir et stocker que la part du flux veineux de retour à proportion du temps d'obturation de la systole, de telle sorte que, si, du fait de cet effet relatif et proportionnel, les atriums C1 et C2 acceptent de s'ouvrir au flux sanguin veineux de retour en attente de la phase de la diastole active, ils n'acceptent cependant que le volume désirable et ne peuvent pas soutirer le circuit veineux en jouant le rôle intempestif de pompe aspirante qui aurait l'effet catastrophique de déprimer les veines du circuit du retour sanguin.

Accessoirement, la prise en charge des sangs artériels et leur adduction vers les appendices (2, 3) de connexion extérieurs au travers de l'espace volumétrique D nécessitent deux conduits intérieurs (8, 9), en double cloison rabattue de la coque de telle sorte que ce dispositif secondaire, contienne et conduise le sang vers les raccords (2, 3) des adducteurs naturel tout en assurant l'étanchéité des espaces volumétriques A1 et A2 par rapport à l'espace volumétrique D.

Cet espace volumétrique D accueille, comme il a été dit, les deux tubulures élastiques (49, 50), identiques, de forme irrégulière, adaptée, pour exploitation maximale du volume d'extension disponible dans les espaces C1 et C2 immergés dans ledit espace pneumatique D.

L'espace volumétrique variable des espaces C1 et C2 constitue le quatrième type d'espace volumétrique, caractérisé en ce que C1 et C2 reçoivent les sangs veineux, en attente d'admission ventriculaire diastolique, et acceptent de les stocker par dilatation contrôlée, menée, et synchronisée, par la systole, et, par le fait, assurent la fonction des atriums.

Les tubulures (49, 50) sont solidaires de la cloison d'isolement (6) et s'ouvrent sur les espaces B1 et B2 par les lumières circulaires (29, 30) ci-avant décrites. Elles sont également solidaires des veines caves et pulmonaires par les appendices de raccordement (4,5) aux adducteurs veineux naturels étant entendu que la représentation qui en est faite par les figures est purement schématique et ne rend pas compte de leurs forme ni de leur disposition pratique dans le cadre d'une réalisation anatomique.

Accessoirement, il convient de concevoir les caractéristiques de forme et d'épaisseur des tubulures élastiques (49, 50) de telle sorte que le volume mort des tubulures additionné de la moitié du volume systolique maximum soit acquis en fin de systole, dans le cas de leurs dilatation maximum, dans les limites des parties rigides, disponibles, de l'espace D, sans que celles-ci s'opposent à la dilatation des atriums C1 et C2 et sans que les tubulures (49, 50) entrent en contact surfacique avec leur environnement. Etant précisé que pour un gain de place et une bonne exploitation de l'espace d'expansion disponible, l'espace volumétrique mort des dites tubulures doit être le plus petit possible.

Accessoirement, l'organisation générale nécessite l'insertion de moyens de communication à savoir, des valves anti-retour entre les atriums C1 et C2 et les ventricules A1 et A2 ainsi qu'entre les lumières d'éjection des ventricules et les appendices de connexion aux artères naturelles.

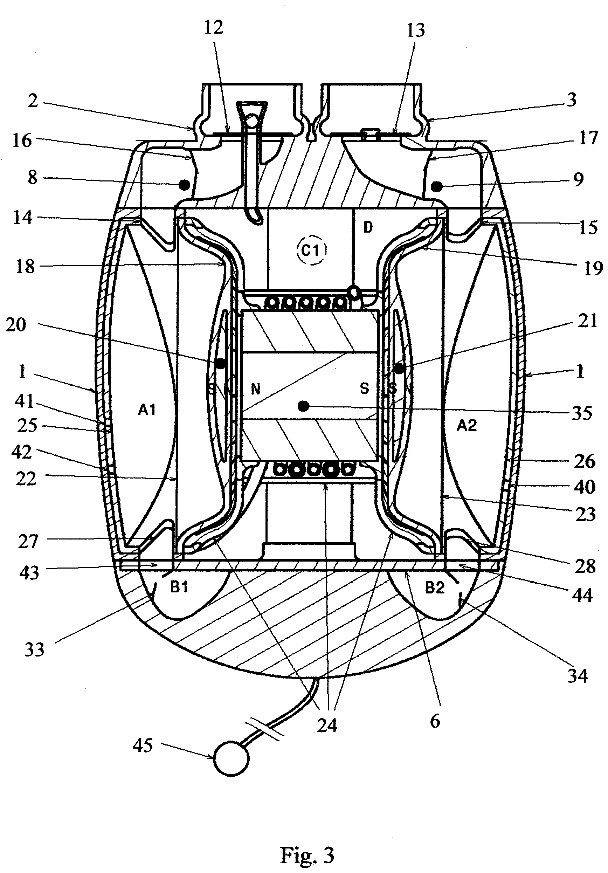

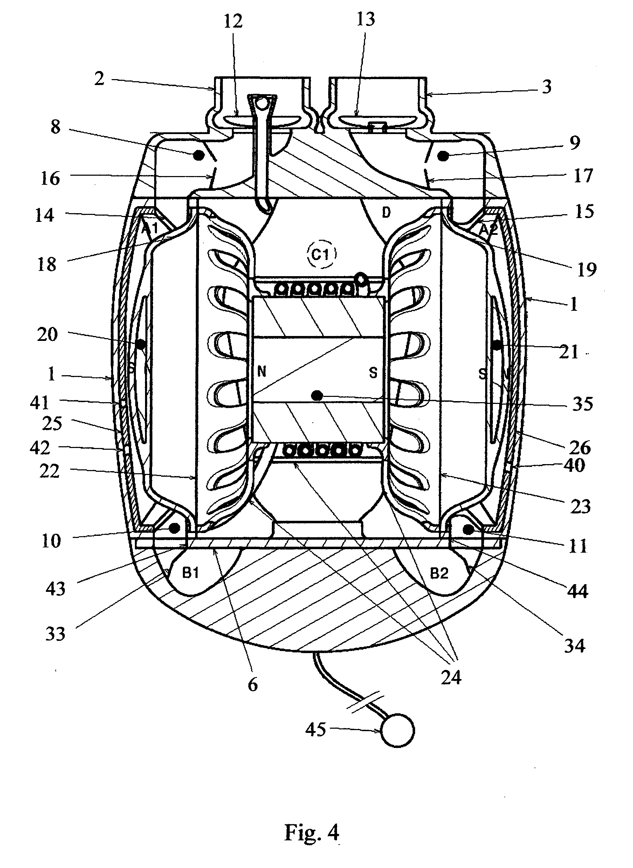

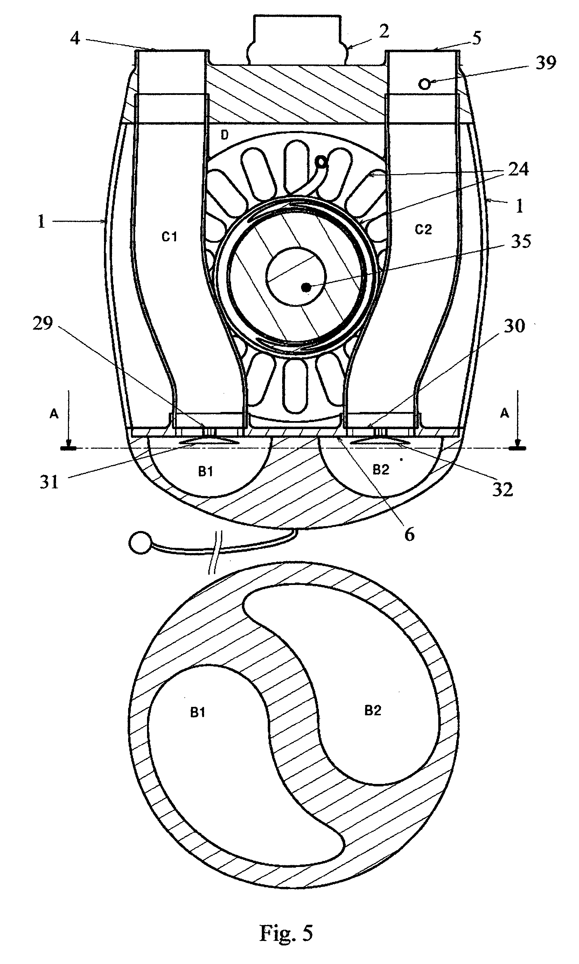

Les valves sont de deux types distincts comme il ressort des figures 3, 4, 5, 7 et 8.

Le premier type de valve est classique, constitué d'un voile circulaire élastique, en élastomère (54), au centre duquel s'élève un appendice de fixation cylindrique, perpendiculaire à son centre, lequel est prisonnier d'une bague concentrique, solidaire d'un anneau périphérique par trois branches de section hydrodynamiques, lequel anneau, la bague et les trois branches de liaison, constituent le support de fixation et de présentation, (46) du voile élastique (54), de telle façon que le dit voile s'élève et retombe sur la tranche de l'anneau de son support de présentation qui fait fonction de siège circulaire (46), lequel support est installé dans les lumières circulaires (29, 30) de la cloison d'isolement (6) pour isoler les atriums C1 et C2 des espaces de transit B1 et B2, ainsi que dans les appendices de connexion (2, 3) des artères naturelles pour la chasse systolique.

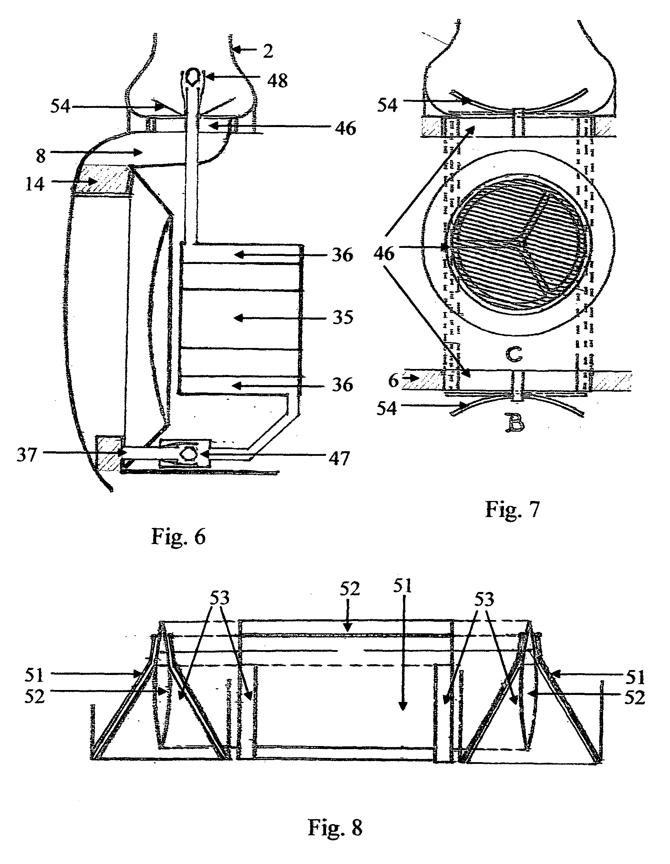

Le deuxième type (16, 17, 33, 34) qui se déduit du caractère oblong des lumières des jupes des espaces A1 et A2, est constitué de deux lames (51) élastiques, en élastomère, selon la figure 8, lesquelles lames se referment, d'un même mouvement, sur une lame d'appui (52) rigide, médiane, maintenue et présentée par deux triangles (53), avec lesquels ladite lame d'appuis forme chevalet, dispositif qui autorise une grande laxité des dites lames élastiques.

Ces valves lamellaires, selon les figures 3, 4 et 8, sont installées en avant des lumières d'admission oblongues (27, 28) pour les sangs veineux dans les espaces B1 et B2, et en avant des appendices de raccordement artériel (2, 3) et de leurs valves circulaires (12, 13) dans les conduits d'exutoires artériels (8, 9) pour le sang systolique artériel.

La surface de la section du tronc aortique pour un individu adulte de taille moyenne et de poids normal est communément admise pour valoir cinq centimètres carré.

En vertu de cette contingence, il est souhaitable que toutes les lumières de communication offrent une surface de section égale ou légèrement supérieure à cinq centimètres carré. L'organisation proposée et revendiquée réserve la possibilité de satisfaire à est impératif.

En outre, il convient de disposer des barorécepteurs (39, 40) sur les parties dures de la cavité ventriculaire A2 et de l'atrium C2 du demi-coeur gauche, connectés à un dispositif électronique de pilotage (45) de l'électro-aimant (35) qui fait fonction, d'une part, de commutateur, primaire, pour ouvrir, seulement, le circuit électrique, servant, de l'étectro-aimant (35), et d'autres parts, étant également connecté à des capteurs de gaz carbonique (41) et d'adrénaline (42), implantés sur la face interne de la paroi de la coque du ventricule A1 du demi-coeur-droit, ledit dispositif électronique de pilotage fait fonction de modulateur de l'impulsion électrique délivrée à l'électro-aimant en durée et en intensité selon les taux de gaz carbonique et d'adrénaline constatés grâce aux capteurs précités.

Le dispositif électronique de pilotage (45) doit être détaché en extérieur du dispositif revendiqué et ramené sous la peau pour faciliter, sans intervention chirurgicale, les manipulations de réglage, de programmation et d'ajustements éventuels.

En outre, le fonctionnement de l'électro-aimant (35) implique un effet joule intempestif qui conduit à organiser un refroidissement accessoire pour disperser les calories produites par le solénoïde.

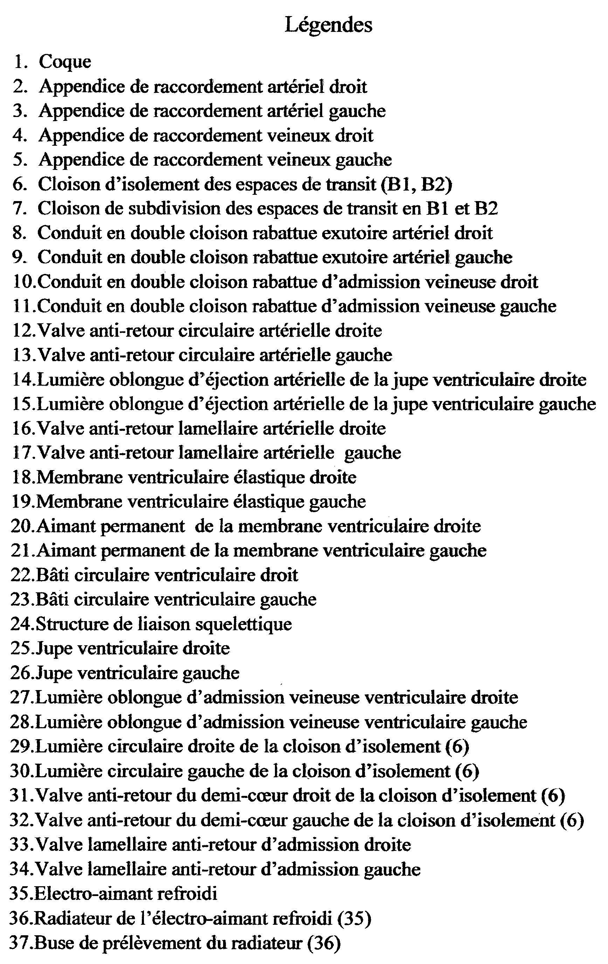

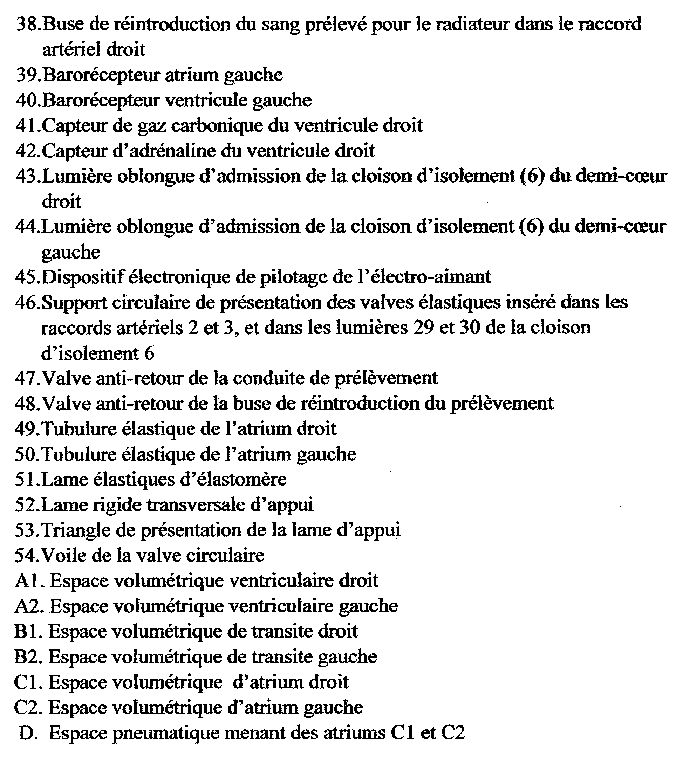

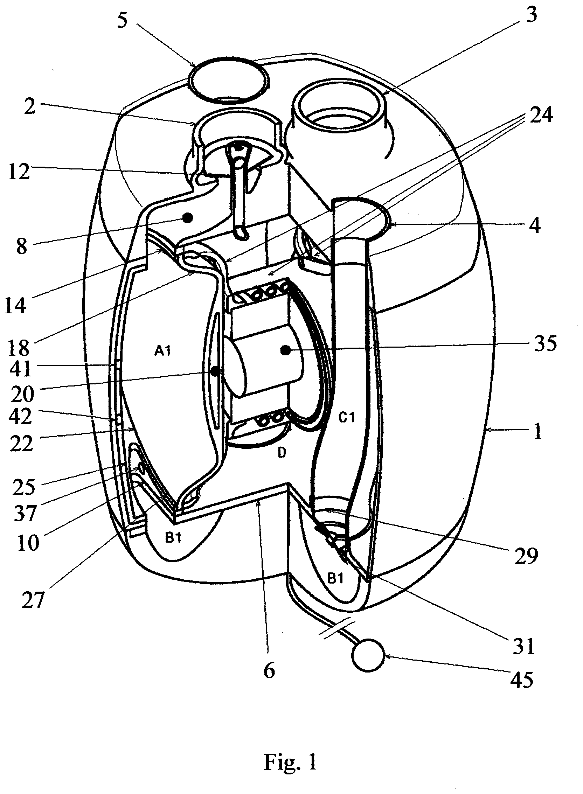

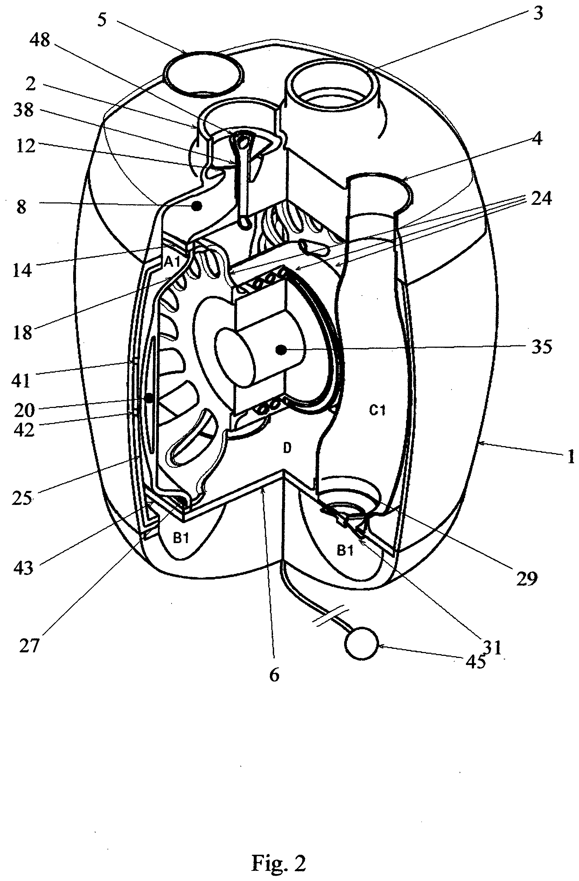

Pour ce faire un petit pourcentage de sang veineux doit être détourné dans le demi-coeur droit, par une buse hydraulique (37), équipée d'une valve anti-retour, laquelle buse est ouverte dans le conduit d'admission du ventricule droit (10), lequel sang détourné est dirigé vers un radiateur périphérique (36) de l'électro-aimant (35) et reconduit pour être réintroduit dans l'appendice de raccordement (2) du demi-coeur droit, en aval de sa valve circulaire (12), par une buse de réintroduction (38), équipé d'une valve anti-retour et installée en venturi, selon les figures 1, 2, 3, 4 et 6.

### Fonctionnement :

Après raccordement aux adducteurs naturels et purge de l'ensemble du dispositif revendiqué,
Au repos, les ventricules sont pleins et prêts pour la systole. A ce stade l'électro-aimant (35) n'est pas alimenté. Le fonctionnement peut alors commencer.

Pour ce faire les barorécepteurs (39, 40) étant équilibrés par couple dans le demi-coeur gauche, le dispositif électronique de pilotage (45) est autorisé à délivrer et moduler une impulsion électrique appropriée aux besoins du moment en durée et en intensité, en fonction des taux de gaz carbonique et d'adrénaline pour la mise en oeuvre de l'électro-aimant (35).

### Systole :

L'impulsion électrique délivrée dans le solénoïde de l'électro-aimant (35) induit la magnétisation et la polarisation du noyau de fer doux.

Les disques d'aimant permanent (20, 21) inclus dans les membranes (18, 19), précédemment rappelés au centre par le fer doux, inerte, en fin de diastole, sont opposés aux pôles de l'électro-aimant comme il est dit dans la description, de telle sorte que les aimants permanents sont repoussés vers l'extérieur.

Les membranes (8, 9) sont dès lors projetées vers la coque dans les espaces ventriculaires A1 et A2, pour la chasse spasmodique systolique.

La course, variable, des membranes, détermine le volume variable de la chasse systolique.

Ce déplacement des membranes, qui induit une diminution du volume ventriculaire et donc une mise en pression du sang dans les espaces ventriculaires A1 et A2, pour la chasse du volume systolique vers les artères.

Induit dans le même temps, et parallèlement, un mouvement de retrait dans l'espace D, de telle sorte que ce mouvement des membranes qui provoque une diminution de l'espace volumétrique de A1 et A2 provoque une augmentation équivalente, compensatoire, du volume de l'espace pneumatique D.

Cette augmentation de volume augmente la dépression pneumatique de l'espace volumétrique D, laquelle dépression agit sur les parois des atriums C1 et C2 pour les affaiblir et donc les distendre. Cette distension combinée avec la pression constante du flux sanguin de retour augmente le volume des atriums qui dés lors acceptent le flux de sang veineux, pour stockage temporaire synchrone, sans interruption du flux retour du sang.
Dés la fin de la systole, telle que programmée par le dispositif électronique de pilotage (45), l'impulsion électrique est interrompue.

### Diastole :

Le solénoïde n'est plus alimenté. L'électro-aimant (35) est désactivé.

Le noyau de fer doux n'étant plus polarisé, ce dernier, fixe, rappelle les aimants permanents (20, 21) des membranes (18, 19) qui en outre se rappellent entre eux du fait que leurs pôles en vis-à-vis sont différents, comme il a été dit dans la description.

Ce rappel magnétique passif, combiné avec la dépression pneumatique active de l'espace volumétrique D, qui joue le rôle de ressort de rappel diffus, additionné de la relaxation de la distension des tubulures (49, 50) des atriums C1 et C2, qui joue également un effet ressort de rappel, induit le retrait des membranes ventriculaires (18, 19) et par conséquent une dépression dans les espaces A1 et A2, en même temps qu'une augmentation de pression dans les atriums C1 et C2.

Cette combinaison de forces induit, dans un premier temps, la purge du sang stocké vers les espaces ventriculaires A1 et A2, en état de dépression, du fait du retrait des membranes ventriculaires (18, 19), via les espaces de transit B1 et B2, de telle sorte que la purge sous pression des espaces C1 et C2 étant aspirée par la dépression ventriculaire, la surpression des atriums passe inaperçu du système sanguin veineux de retour, en amont des atriums C1 et C2, et ne modifie pas la pression du flux retour, en amont, dans le réseau veineux.

A l'issue de cette phase initiale de la diastole, l'état de dépression de l'espace pneumatique D est revenu à la normale et le sang en retour, en flux continu, fini de remplir les espaces ventriculaires A1 et A2, via les espaces B1 et B2 jusqu'à ce que les membranes (18, 19) soient revenues à leurs position de retrait maximum.

A ce stade les pressions constatées dans le ventricule A2 du demi-coeur gauche et dans l'atrium C2 du demi-coeur gauche parviennent au point d'équilibre.

Le couple de barorécepteurs (39, 40) constate l'équilibre barométrique et autorise le dispositif électronique de pilotage (45) à délivrer l'impulsion électrique suivante, modulée en durée et en intensité par ce dernier en fonction des autres paramètres (gaz carbonique et adrénaline). Le cycle est accompli.

Les efforts des demi-coeurs gauche et droit se développent de façon symétrique et opposée de parts et d'autres du centre de gravité du dispositif ; l'ensemble est théoriquement stable sur lui-même, et est, par conséquent, extérieurement inerte à l'intérieur de la cavité péricardique dans la cage thoracique, ce qui n'est pas sans intérêt au regard des réactions inflammatoires intempestives, potentielles, que la logique commande d'éviter autant que faire se peut.

En outre, il convient de préciser que pour une réalisation pratique et pour une mise en oeuvre effective du dispositif de coeur artificiel revendiqué, il est impératif que tous les matériaux utilisés soient hémocompatible, pour leurs partiess en contact avec le sang du receveur, et, à tout le moins biocompatible, pour leurs parties en contact avec le reste de l'organisme.

## Revendications

1. Dispositif de coeur artificiel intégral implantable dans la cavité péricardique, comprenant deux ventricules droit (A1) et gauche (A2) à pulsion simultanée, et leurs atriums réactifs droit (C1) et gauche (C2), immergés dans un espace pneumatique à dépression variable D, lesquels sont menés, en opération, par un électro-aimant (35), agissant séquentiellement par répulsion sur des disques d'aimant permanent (20, 21) inclus dans des membranes élastiques ventriculaires (18, 19), qui battent simultanément dans les espaces ventriculaires A1 et A2, et, en sens contraire, dans l'espace pneumatique D qui héberge des tubulures élastique en qualité d'atriums, ledit dispositif permettant d'éjecter les volumes systoliques simultanément, et accepte de stocker dans les atriums la proportion des sangs veineux en retour permanent, pendant la systole, de telle sorte que ledit retour permanent n'est pas interrompu par l'obturation séquentielle systolique des lumières d'admissions, ledit dispositif **caractérisé en ce qu'**il est constitué d'une coque rigide (1), subdivisée en six espaces volumétriques hydrauliques A1, A2, B1, B2, C1, C2, et un espace volumétrique pneumatique D, et, à l'intérieur de laquelle coque est installée solidairement une structure transversale constituée par les jupes ventriculaires (25, 26), les bâtis (22, 23) et une structure squelettique (24), intermédiaire de liaison, laquelle maintient les membranes ventriculaires (18, 19) et les aimants permanents inclus (20, 21), et supporte entre les dites membranes un électro-aimant refroidi (35), lequel en interaction avec les aimants (20, 21) constitue le dispositif électromagnétique d'animation générale, lesquelles dites membranes ventriculaires (18,19), les parties rigides de la coque (1) et les jupes ventriculaires (25, 26) délimitent au centre de la coque (1), l'espace volumétrique pneumatique D, dans l'ambiance pneumatique duquel sont immergées les tubulures élastiques (49, 50) des espaces hydrauliques d'atrium (C1, C2), tandis que les mêmes membranes (18, 19), les jupes (25, 26), et la coque (1) constituent les espaces volumétriques, hydrauliques, ventriculaires (A1, A2), lequel dispositif comprend des valves anti-retour (31, 32, 33, 34) entre les atriums (C1, C2) et les ventricules (A1, A2), des valves anti-retour (12, 13, 16, 17) entre les ventricules (A1, A2) et leurs exutoires artériels (2, 3), des capteurs de gaz carbonique (41) et d'adrénaline (42) ainsi que des barorécepteurs (39, 40), connectés à un dispositif électronique interactifs de pilotage (45), détaché en extérieur et comprend en accessoire indispensable, un radiateur de refroidissement (36) de l'électro-aimant (35).

2. Dispositif selon la revendication 1, constitué par une coque rigide, destiné à isoler le dispositif du reste de l'organisme et à servir de squelette pour l'ancrage intérieur des dispositifs concourants, **caractérisé en ce que** ladite coque rigide (1), de type ovoïde, comporte, de première part, en extérieur, des appendices de communication pour le raccordement aux adducteurs naturels, deux pour les artères (2, 3), équipés de valves anti-retour élastiques (12, 13), et deux, au moins, pour les veines (4, 5), et de deuxième part à l'intérieur de ladite coque, une combinaison de cloisons concourantes, l'une (6), qui isole les espaces, hydrauliques, spécifiques, de transit et de retournement B, laquelle, comporte deux lumières, circulaires, d'accès (29, 30), équipées de valves anti-retour (31, 32), ainsi que deux lumières, oblongues, de sortie (43, 44), et l'autre cloison (7), de subdivision, dont le plan de symétrie est perpendiculaire à la cloison d'isolement (6), de telle sorte que, à l'opposé des appendices précités (2, 3), ladite combinaison isole deux fosses de transit et de retournement hydraulique, symétriques (B1, B2), ladite coque comporte également quatre conduits en double cloison rabattue, deux, l'un (10) pour le demi-coeur droit, et l'autre (11) pour le demi-coeur gauche, de telle sorte que ces deux conduits canalisent les flux sanguins veineux des espaces hydraulique de transit (B1, B2), via les lumières oblongues (43 et 44) de la cloison (6), vers les lumières d'admissions oblongues (27, 28) des espaces hydrauliques, ventriculaires (A1, A2), lesquels conduits sont équipés de valves lamellaires (33, 34), et deux autres, l'un (8), pour le demi-coeur droit et l'autre (9), pour le demi-coeur gauche, de telle sorte que ces conduits canalisent les flux sanguins des lumières d'éjection (14, 15) des espaces hydrauliques, ventriculaires (A1, A2) vers les exutoires artériels, lesquels conduits (9, 10) sont équipés de valves lamellaires anti-retour (16, 17), laquelle coque comporte également sur sa paroi intérieure les moyens d'ancrage en vis-à-vis, propres à recevoir et fixer une structure transversale complémentaire.

3. Dispositif selon les revendications 1 et 2, constitué d'une structure transversale qui complète l'architecture intérieure avec la coque (1), **caractérisée en ce que** la dite structure comporte, depuis les ancrages de la paroi intérieure de la coque (1), les deux jupes ventriculaires (25, 26), solidaires des bâtis de maintien (22, 23), lesquels bâtis sont liés entre eux dos-à-dos, par l'intermédiaire d'une structure squelettique de liaison (24), de telle sorte que la dite structure complémentaire maintienne et présente les membranes (18, 19) et leurs disques, inclus, d'aimant permanent (20, 21), ainsi qu'un électro-aimant refroidi intercalaire (35).

4. Dispositif selon les revendications 1, 2 et 3, hydraulique, de pompe ventriculaire, **caractérisé en ce que** les membranes ventriculaires (18, 19) sont constituées d'un voile d'élastomère préformé à l'intérieur et au centre duquel est inclus un disque d'aimant permanent, (20, 21), noyé dans l'élastomère des dites membranes (18, 19), lesquelles membranes sont maintenues à leurs périphérie par les bâtis de maintien (22, 23), prolongés par les jupes (25, 26), ancrées sur la paroi de la coque (1), de telle sorte que cette combinaison de moyens enferme les espaces hydrauliques, ventriculaires (A1, A2), lesquelles membranes battent dans lesdits espaces (A1, A2) qui comportent, ouvert dans les jupes (25, 26), des lumières oblongues, d'admission (27, 28), servies par les conduits d'admission veineuse (10, 11), ainsi que des lumières oblongues d'éjection artérielle (14, 15) servant les conduits exutoire artériels (8, 9).

5. Dispositif selon les revendications 1, 3 et 4, électromagnétique, menant, en opération, d'animation générale, **caractérisé en ce que** la structure transversale (24) maintient et présente les membranes ventriculaires (18, 19) et leurs aimants permanents inclus (20, 21) au moyen des bâtis (22, 23) de telle sorte que le pôle nord de l'un regarde le pôle sud de l'autre, et que la structure de liaison (24) maintient un électro-aimant refroidi intercalé entre les deux aimants permanents inclus (20, 21), de telle sorte que son pôle nord s'oppose au pôle nord intérieur de l'un des aimants tandis que son pôle sud s'oppose au pôle sud intérieur de l'autre aimant permanent, lequel dispositif induit, par répulsion magnétique, le battement mené des membranes, solidaires des aimants inclus, simultanément dans les espaces ventriculaires (A1, A2) et dans l'espace volumétrique pneumatique D, de telle sorte que, durant la systole, ledit battement comprime les espaces volumétriques hydrauliques ventriculaires (A1, A2), et simultanément, déprime l'espace volumétrique pneumatique D, et inversement, durant la diastole.

6. Dispositif selon les revendications 1 et 5, mené, en opération, d'animation pneumatique menant les atriums immergés, **caractérisé en ce que**, déduction faite des espaces volumétriques hydrauliques (A1, A2, B1, B2, C1, C2), et des conduits (8, 9, 10, 11) faisant saillie dans la coque (1), l'espace volumétrique pneumatique D restant, à volume variable, qui sépare les membranes ventriculaires (18, 19) et qui héberge l'électro-aimant refroidi (35), est, le dit espace pneumatique D, occupé par un gaz fonction de sa densité et de son coefficient d'élasticité en état de légère dépression durant sa relaxation en fin de la diastole, de telle sorte, que les membranes (18, 19) battent simultanément dans les espaces volumétriques ventriculaires (A1, A2), et dans l'espace volumétrique pneumatique D, en sens contraire, et que de ce fait, l'ambiance pneumatique du dit espace volumétrique pneumatique D est à dépression variable, menée, du fait de l'élasticité du gaz d'occupation, soumis à l'action des dites membranes.

7. Dispositif selon les revendications 1 à 6, hydraulique, mené, en opération, d'atrium C1, C2, **caractérisé en ce que** les dits atriums sont constitués de tubulures élastiques, préformées, irrégulières, identiques, enveloppantes et symétriques, qui joignent les appendices de connections (4, 5) de la coque (1) aux espaces hydrauliques de transit et de retournement (B1, B2), par les lumières (29, 30) ouvertes dans la cloison d'isolement (6), lesquelles tubulures sont immergées dans l'ambiance pneumatique à dépression variable de l'espace pneumatique D, de telle sorte que, les variations de la dépression pneumatique, dans l'espace D, agissent sur l'élasticité des parois des tubulures (49, 50), qui enferment les espaces hydrauliques d'atrium (C1, C2), pour induire la variation de l' élasticité des dites tubulures, ainsi que leur expansion subséquente sous l'action de la pression interne du sang veineux qui les occupe et leur permettre d'accepter les dits sangs veineux, en retour, pour stockage temporaire, pendant la durée de la systole.

8. Dispositif selon les revendications 1 et 5, comportant un dispositif de refroidissement de l'électro-aimant (35) en relation avec le demi- coeur droit, à savoir, le demi-coeur servant des poumons, **caractérisé en ce qu'**il comporte un radiateur (36), périphérique à l'électro-aimant (35), lequel radiateur est parcouru, en opération, par une petite proportion de sang détourné de l'espace ventriculaire A1 du demi- coeur droit, pendant la durée de la systole, au moyen d'une buse (37) équipée d'une valve anti-retour (47), ménagée, ladite buse, dans toute partie dure de la jupe (25) ou dans le conduit d'admission (10), comme repéré sur la figure 1, laquelle buse est raccordée au radiateur (36) par une conduite d'adduction équipée d'une valve anti-retour (47), lequel volume de sang prélevé est reconduit vers l'exutoire artériel (2) du demi-coeur droit par une conduite qui se termine par une buse équipée d'une valve anti-retour (48), disposée en *venturi* dans le dit exutoire artériel, en aval de la valve anti-retour principale (12).

9. Dispositif selon les revendications 1 et 2, comportant un dispositif de communication anti-retour, à savoir la valve circulaire qui équipe les appendices de la coque (2, 3) et les lumières (29, 30) de la cloison d'isolement (6), **caractérisé en ce que** la dite valve anti-retour est constituée d'un voile circulaire (54), élastique, en élastomère, au centre duquel s'élève un appendice solidaire, de fixation, cylindrique, perpendiculaire à son plan, lequel est prisonnier d'une bague solidaire d'un anneau périphérique, concentrique, par le moyen de trois branches, de section hydrodynamiques, lequel anneau, les branches de liaison et la bague précités, constituent le support de fixation et de présentation (46) du voile élastique (54), de telle façon que le dit voile s'élève et retombe sur la tranche de l'anneau de son support de présentation qui fait fonction de siège d'obturation circulaire, lequel support est installé dans les lumières (29, 30) de la cloison (6), ainsi que dans les appendices de raccordement artériels (2, 3).

10. Dispositif selon les revendications 1 et 2, comportant un dispositif de communication anti-retour, à savoir, la valve lamellaire, installées dans les conduits (8, 9, 10, 11), **caractérisé en ce que** ladite valve est constituée de deux lames élastiques (51), en élastomère, qui s'appuient en fermeture sur une lame rigide de section hydrodynamique, comportant un bord d'attaque et un bord de fuite propre à éviter les turbulences, laquelle lame (52) est présentée aux bords de fuite des dites lames élastiques par des triangles (53) avec laquelle ils forment chevalet et sur les tranches desquels triangles les bords de fuite latéraux des dites lames élastiques s'appuient pour fermer le dispositif d'obturation durant le temps de la systole, en complément des valves circulaires.

11. Dispositif selon les revendicatiions 1, 4, 5 et 6, comportant un dispositif électronique primaire de commutation initiale de l'impulsion électrique, **caractérisé en ce qu'**il comporte deux barorécepteurs (39, 40) installés sur les parties dures du demi-coeur gauche, dans la face interne de la coque (1),comprise dans l'espace ventriculaire A2 pour l'un (40), et dans les patries dures de l'espace d'atrium C2 du même demi-coeur gauche pour l'autre (39), de telle sorte que couplés avec le dispositif électronique de pilotage (45) de l'électro-aimant, ce dit dispositif constate l'équilibre des pressions du moment et autorise la délivrance de l'impulsion électrique nécessaire à l'électro-aimant (35) pour le début, seulement, de la systole.

12. Dispositif selon les revendications 1, 4, 5, 6 et 11, comportant un dispositif électronique de modulation et d'interruption de l'impulsion électrique de l'électro-aimant (35), **caractérisé en ce que** le dispositif électronique de pilotage (45), détaché en extérieur sous la peau, est connecté à des capteurs de gaz carbonique (41) et d'adrénaline (42) installés sur la surface interne de la coque (1) dans l'espace ventriculaire A1 du demi- coeur droit, de telle sorte que, en fonction des taux constatés de gaz carbonique et d'adrénaline, le dit dispositif électronique de pilotage module l'intensité et la durée de l'impulsion électrique nécessaire à la systole du moment et que la durée, ainsi programmée, ayant atteint son terme, il ferme le circuit électrique ouvert par le commutateur électronique primaire précité, avec lequel dispositif il est également couplé, pour agir en synergie synchronisée.

## Patentansprüche

1. Integrale künstliche Herzvorrichtung, die in die Perikardhöhle implantierbar ist, umfassend zwei Herzventrikel, einen rechten (A1) und linken (A2) mit simultanem Impuls, und deren reaktiven Atrien, ein rechtes 5 (C1) und linkes (C2), die in einem pneumatischen Raum mit variablem Unterdruck D eingetaucht sind, welche im Betrieb durch einen Elektromagneten (35) geführt werden, welcher sequenziell durch Abstoßung auf Dauermagnetscheiben (20,21) einwirkt, die in elastischen ventrikulären Membranen (18,19) beinhaltet sind, die gleichzeitig in den ventrikulären Räumen A1 und A2 schlagen, und, in entgegengesetzter Richtung, in dem pneumatischen Raum D, der elastische Stutzen als Atrien beherbergt, wobei es die Vorrichtung erlaubt, die systolischen Volumina gleichzeitig auszustoßen, und es akzeptiert, in den Atrien den Anteil von venösem Blut im permanenten Rückfluss währen der Systole derart zu speichern, dass der permanente Rückfluss durch den sequenziellen systolischen Verschluss der Einlassöffnungen nicht unterbrochen wird, wobei die Vorrichtung **dadurch gekennzeichnet ist, dass** sie aus einer starren Schale (1) gebildet wird, die in sechs hydraulische volumetrische Räume A1, A2, B1, B2, C1, C2, und einen pneumatischen volumetrischen Raum D unterteil wird, und, im Inneren der Schale feststehend eine querlaufende Struktur installiert ist, die durch die ventrikulären Schürzen (25, 26), die Gehäuse (22,23) und eine zwischenliegende verbindende Skelettstruktur (24) gebildet wird, welche die ventrikulären Membranen (18,19) und die beinhalteten Dauermagneten (20, 21) festhält, und zwischen den Membranen einen gekühlten Elektromagneten (35) trägt, welcher in Zusammenwirkung mit den Magneten (20, 21) die allgemeine elektromagnetische Animationsvorrichtung bildet, wobei die ventrikulären Membranen (18,19), die starren Abschnitte der Schale (1) und die ventrikulären Schürzen (25,26) in der Mitte der Schale (1) den pneumatischen volumetrischen Raum D eingrenzen, in dessen pneumatischer Atmosphäre die elastischen Stutzen (49, 50) der hydraulischen Atrienräume (C1, C2) eingetaucht sind, während dieselben Membrane (18,19), die Schürzen (25,26), und die Schale (1) die hydraulischen, ventrikulären volumetrischen Räume (A1, A2) bilden, wobei die Vorrichtung Rückschlagventile (31, 32, 33, 34) zwischen den Atrien (C1, C2) und den Herzventrikeln (A1, A2) umfasst, Rückschlagventile (12, 13, 16, 17) zwischen den Herzventrikeln (A1, A2) und deren arteriellen Ausflüssen (2, 3), Sensoren für Kohlendioxid (41) und für Adrenalin (42), sowie Druckrezeptoren (39,40), die mit einer interaktiven elektronischen Steuerungsvorrichtung (45), die nach außen ausgelagert ist und als unverzichtbares Zubehör, einen Kühlradiator (36) für den Elektromagneten (35) umfasst, verbunden sind.

2. Vorrichtung nach Anspruch 1, die durch eine starre Schale gebildet wird, die dazu bestimmt ist, die Vorrichtung vom Rest des Organismus zu trennen, und als Skelett zur inneren Verankerung der zusammenwirkenden Vorrichtungen zu dienen, **dadurch gekennzeichnet, dass** die starre Schale (1), in der Art einer Eiform, erstens, außen, Kommunikationsanhänge für den Anschluss an die natürlichen Adduktoren beinhaltet, zwei für die Arterien (2, 3), die mit elastischen Rückschlagventilen (12,13) ausgestattet sind, und mindestens zwei, für die Venen (4,5), und zweitens, im Inneren der Schale, eine Kombination von zusammenwirkenden Wänden, die eine (6), welche die spezifischen, hydraulischen Räume für den Transit und den Rückfluss B trennt, welche zwei kreisförmige Öffnungen für den Zugang (29, 30) beinhaltet, die mit Rückschlagventilen (31,32) ausgestattet sind, sowie zwei längliche Löcher für den Austritt (43,44), und die andere Wand (7), zur Unterteilung, deren Symmetrieebene senkrecht zur Trennwand (6) verläuft, sodass die Kombination gegenüber den zuvor genannten Anhängen (2,3) zwei symmetrische hydraulische Transit- und Rückflussgräben (B1, B2) isoliert, wobei die Schale ebenfalls vier Leitungen mit umgeklappter Doppelwand beinhaltet, zwei, die eine (10) für die rechte Herzhälfte, und die andere (11) für die linke Herzhälfte, sodass diese beiden Leitungen den Venenblutstrom der hydraulischen Transiträume (B1, B2) über die länglichen Öffnungen (43 und 44) von der Wand (6) zu den länglichen Einlassöffnungen (27,28) der hydraulischen ventrikulären Räume (A1, A2) kanalisiert, wobei die Leitungen mit Lamellenventilen (33,34) ausgerüstet sind, und zwei andere, die eine (8) für die rechte Herzhälfte und die andere (9) für die linke Herzhälfte, sodass die Leitungen die Blutströme von den Ausstoßöffnungen (14, 15) der hydraulischen ventrikulären Räume (A1, A2) zu den arteriellen Ausflüssen kanalisieren, wobei die Leitungen (9,10) mit Lamellen-Rückschlagventilen (16, 17) ausgerüstet sind, wobei die Schale auf ihrer inneren Wand auch Mittel zum Verankern gegenüber beinhaltet, die dafür geeignet sind, eine ergänzende querlaufende Struktur aufzunehmen und zu fixieren.

3. Vorrichtung nach den Ansprüchen 1 und 2, die aus einer querlaufenden Struktur gebildet wird, welche die interne Architektur mit der Schale (1) vervollständigt, **dadurch gekennzeichnet, dass** die Struktur aus den Verankerungen der Innenwand der Schale (1) heraus die beiden ventrikulären Schürzen (25, 26) beinhaltet, die fest mit den Haltgehäusen (22, 23) verbunden sind, wobei die Gehäuse Rücken an Rücken über eine verbindende Skelettstruktur (24) miteinander verbunden sind, sodass die ergänzende Struktur die Membrane (18, 19) und deren Scheiben, inklusive Dauermagnet (20, 21), sowie einen gekühlten Zwischenlagen-Elektromagneten (35) festhält und aufweist.

4. Hydraulische Vorrichtung nach den Ansprüchen 1, 2 und 3, aus einer ventrikulären Pumpe, **dadurch gekennzeichnet, dass** die ventrikulären Membranen (18,19) aus einem vorgeformten Elastomer-Segel gebildet ist, in dessen Inneren und Mitte eine Dauermagnetscheibe (20, 21) eingeschlossen ist, die in dem Elastomer der Membrane (18,19) eingetaucht ist, wobei die Membrane an ihren Umfängen durch die Haltegehäuse (22, 23) gehalten werden, die durch die Schürzen (25, 26) verlängert werden, die an der Wand der Schale (1) verankert sind, sodass diese Kombination von Mitteln die hydraulischen ventrikulären Räume (A1, A2) einschließt, wobei die Membrane in den Räumen (A1, A2) schlagen, welche in die Schürzen (25,26) offene längliche Einlassöffnungen (27, 28) beinhalten, die durch die venösen Einlassleitungen (10, 11) bedient werden, sowie längliche arterielle Ausstoßöffnungen (14, 15), die die arteriellen Ausflussleitungen (8,9) bedienen.

5. Elektromagnetische Vorrichtung nach den Ansprüchen 1, 3 und 4, welche im Betrieb die allgemeine Animation führt, **dadurch gekennzeichnet, dass** die querlaufende Struktur (24) die ventrikulären Membranen (18, 19) und deren beinhalteten Dauermagneten (20, 21) anhand der Gehäuse (22, 23) derart hält und aufweist, dass der Nordpol des einen dem Südpol des anderen zugewandt ist, und dass die Verbindungsstruktur (24) einen gekühlten, zwischen den beiden beinhalteten Dauermagneten (20, 21) eingesetzten Elektromagneten festhält, sodass sein Nordpol von dem inneren Nordpol des einen der Magneten abgewandt ist, während sich sein Südpol von dem inneren Südpol des anderen Dauermagneten abwendet, wobei die Vorrichtung durch magnetisches Abstoßen das geführte Schlagen der Membranen, die mit den beinhalteten Magneten fest verbunden sind, gleichzeitig in den ventrikulären Räumen (A1, A2) und in dem pneumatischen volumetrischen Raum D induziert, sodass der Schlag während der Systole die hydraulischen volumetrischen ventrikulären Räume (A1, A2) komprimiert, und gleichzeitig den pneumatischen volumetrischen Raum D eindrückt, und umgekehrt bei der Diastole.

6. Im Betrieb geführte Vorrichtung nach den Ansprüchen 1 und 5, zur pneumatischen Animation, welche die eingetauchten Atrien animiert, **dadurch gekennzeichnet, dass**, nach Deduktion der hydraulischen volumetrischen Räume (A1, A2, B1, B2, C1, C2), und der Leitungen (8, 9, 10, 11), die in die Schale (1) überstehen, der verbleibende pneumatische volumetrische Raum D mit variablem Volumen, der die ventrikulären Membrane (18, 19) trennt und der den gekühlten Elektromagneten (35) beherbergt, der pneumatische Raum D ist, der durch ein Gas in Abhängigkeit von dessen Dichte und seinem Elastizitätskoeffizienten in einem Zustand eines leichten Eindrucks während seiner Entspannung am Ende der Diastole eingenommen wird, sodass die Membrane (18, 19) gleichzeitig in den ventrikulären volumetrischen Räumen (A1, A2) schlagen, und in dem pneumatischen volumetrischen Raum D, in entgegengesetzter Richtung, und dass die pneumatische Atmosphäre des pneumatischen volumetrischen Raums D aus diesem Grund von variabler Ausdehnung ist, die durch die Elastizität des einnehmenden Gases geführt wird, das der Aktion der Membrane unterworfen wird.

7. Im Betrieb geführte hydraulische Vorrichtung eines Atriums C1, C2 nach den Ansprüchen 1 bis 6, **dadurch gekennzeichnet, dass** die Atrien aus vorgeformten, unregelmäßigen, identischen, umhüllenden und symmetrischen elastischen Stutzen gebildet sind, die mit den Verbindungsanhängen (4, 5) der Schale (1) mit den hydraulischen Transit- und Rückflussräumen (B1, B2) durch die offenen Öffnungen (29, 25, 30) in der Trennwand (6) verbunden sind, wobei die Stutzen in die pneumatische Atmosphäre mit variabler Ausdehnung des pneumatischen Raumes D eingetaucht sind, sodass die Variationen des pneumatischen Eindrucks in dem Raum D auf die Elastizität der Wände der Stutzen (49, 50) wirken, welche die hydraulischen Atrienräume (C1, C2) einschließen, um die Variation der Elastizität der Stutzen, sowie deren folgende Ausdehnung unter Wirkung des Innendrucks des Venenbluts zu induzieren, das sie einnimmt, und es ihnen zu ermöglichen, das Venenblut beim Rückfluss für die vorübergehende Speicherung während der Dauer der Systole zu speichern.

8. Vorrichtung nach den Ansprüchen 1 und 5, eine Kühlvorrichtung des Elektromagneten (35) in Verbindung mit der rechten Herzhälfte beinhaltend, nämlich der Herzhälfte, die den Lungen dient, **dadurch gekennzeichnet, dass** sie einen Radiator (36), umlaufend um den Elektromagneten (35) beinhaltet, wobei der Radiator im Betrieb von einem kleinen Anteil von Blut durchflossen wird, das während der Dauer der Systole von dem ventrikulären Raum A1 der rechten Herzhälfte, anhand einer Düse (37) die mit einem eingebauten Rückschlagventil (47) ausgestattet ist, umgeleitet wird, wobei die Düse im gesamten starren Abschnitt der Schürze (25) oder in der Einlassleitung (10), wie in der Figur 1 dargestellt ist, wobei die Düse durch eine Adduktionsleitung, die mit einem Rückschlagventil (47) ausgestattet ist, an den Radiator (36) angeschlossen ist, wobei das Volumen des entnommenen Bluts durch eine Leitung in den arteriellen Ausfluss (2) der rechten Herzhälfte weitergeleitet wird, die durch eine Düse abgeschlossen wird, die mit einem Rückschlagventil (48) ausgestattet ist, das als *Venturi* in dem arteriellen Ausfluss stromabwärts von dem Hauptrückschlagventil (12) angeordnet ist.

9. Vorrichtung nach den Ansprüchen 1 und 2, die eine Rückschlag-Kommunikationsvorrichtung beinhaltet, nämlich das kreisförmige Ventil, das die Anhänge der Schale (2, 3) und die Öffnung (29, 30) der Trennwand (6) ausstattet, **dadurch gekennzeichnet, dass** das Rückschlagventil aus einem elastischen kreisförmigen Segel (54) aus Elastomer gebildet ist, in dessen Mitte sich ein Anhang zur zylindrischen Fixierung, senkrecht zu seiner Ebene, erhebt, welche von einem Ring eingeschlossen ist, der anhand von drei Ästen fest mit einem konzentrischen Umfangsring mit hydrodynamischem Querschnitt verbunden ist, wobei der Ring, die Verbindungsäste und der Ring, die zuvor genannt wurden, den Fixierungs- und Präsentationshalter (46) des elastischen Segels (54) bilden, sodass sich das Segel erhebt und auf die Kante des Ringes seines Präsentationshalters absenkt, der als kreisförmiger Verschlusssitz fungiert, wobei der Halter in den Öffnungen (29, 30) der Wand (6), sowie in den arteriellen Anschlussanhängen (2, 3) installiert ist.

10. Vorrichtung nach den Ansprüchen 1 und 2, die eine Rückschlag-Kommunikationsvorrichtung, nämlich das Lamellenventil in den Leitungen (8, 9 , 10, 11) installiert, beinhaltet, **dadurch gekennzeichnet, dass** das Ventil durch zwei elastische Klingen (51) aus Elastomer gebildet wird, die sich beim Schließen an einer starren Klinge mit hydrodynamischem Querschnitt anlegen, die eine Vorderkante und eine Hinterkante aufweist die geeignet ist um Turbulenzen zu vermeiden, wobei die Klinge (52) an den Hinterkanten der elastischen Klingen durch Dreiecke (53) präsentiert wird, mit denen sie ein Gestell bilden, und sich die seitlichen Hinterkanten der elastischen Klingen an den Kanten des Dreiecks anlegen, um die Schließvorrichtung während der Dauer der Systole, ergänzend zu den kreisförmigen Ventilen, zu schließen.

11. Vorrichtung nach den Ansprüchen 1, 4, 5 und 6, die eine elektronische Primärvorrichtung zur Erstumschaltung des elektrischen Impulses beinhaltet, **dadurch gekennzeichnet, dass** sie zwei Druckrezeptoren (39, 40) beinhaltet, die an dem starren Abschnitten der linken Herzhälfte an der Innenseite der Schale (1) installiert sind, die in dem ventrikulären Raum A2 für den einen (40), und in den starren Abschnitten des Atrienraums C2 derselben linken Herzhälfte installiert sind, für die andere (39), sodass sie mit der elektronischen Steuervorrichtung (45) des Elektromagneten gekoppelt sind, wobei diese Vorrichtung das Gleichgewicht der Drücke des Moments feststellt und die Abgabe des elektrischen Impulses freigibt, der für den Elektromagneten (35) nur zu Beginn der Systole nötig ist.

12. Vorrichtung nach den Ansprüchen 1, 4, 5, 6 und 11, die eine elektronische Vorrichtung zum Modulieren und Unterbrechen des elektrischen Impulses des Elektromagneten (35) beinhaltet, **dadurch gekennzeichnet, dass** die elektronische Steuervorrichtung (45), die außen unter der Haut ausgelagert ist, mit den Sensoren für Kohlendioxid (41) und Adrenalin (42) verbunden ist, die an der inneren Oberfläche der Schale (1) in dem ventrikulären Raum A1 der rechten Herzhälfte installiert ist, sodass die elektronische Steuervorrichtung je nach festgestellten Raten an Kohlendioxid und Adrenalin, die Intensität und die Dauer des elektrischen Impulses moduliert, die für die Systole des Moments nötig ist, und sie, da die so programmierte Dauer am Ende angelangt ist, den Stromkreis schließt, der durch den zuvor genannten elektronischen Schalter geöffnet wurde, mit dem die Vorrichtung ebenfalls gekoppelt ist, um in synchronisierter Synergie zu handeln.

## Claims

1. An integral artificial heart implantable in the pericardial cavity, comprising a right ventricle (A1) and left ventricle (A2) pulsing simultaneously, and the reactive right atrium (C1) and left atrium thereof are immersed in a pneumatic space having a variable vacuum D, which are driven, into operation, by a solenoid (35), acting sequentially, by repulsion, on the permanent magnet discs (20, 21) included in the elastic ventricular membranes (18, 19), which beat simultaneously in the ventricular spaces A1 and A2, and, in the opposite direction, in the pneumatic space D, which houses elastic tubes acting as atria. The said device is used to simultaneously eject systolic volumes, and accept the proportion of continuously returning venous blood to store in the atria, during the systole, such that the said continuous return is not interrupted by sequential systolic closure of the intake ports. The said device **characterised in that** it is made up of a rigid shell (1), divided into six hydraulic volumetric spaces A1, A2, B1, B2, C1, C2, and a pneumatic volumetric space D, and within the said shell a transverse structure is integrally built made of ventricular skirts (25, 26), frames (22, 23) and a skeletal structure (24), acting as an intermediate link, which holds the ventricular membranes (18,19) and the included permanent magnets (20, 21), and between the said membranes supports a cooled solenoid (35), which, when it interacts with the magnets (20, 21), constitutes the general animated electromagnetic device, where the said ventricular membranes (18,19), rigid parts of the shell (1) and the ventricular skirts (25, 26) define the centre of the shell (1), the pneumatic volumetric space D, where elastic tubes (49, 50) of hydraulic atrium spaces (C1, C2) are immersed in its pneumatic atmosphere, while the same membranes (18, 19), skirts (25, 26) and the shell (1) constitute the volumetric, hydraulic, ventricular spaces (A1, A2), the said device comprises non-return valves (31, 32, 33, 34) between the atria (C1, C2) and the ventricles (A1, A2), non-return valves (12, 13, 16, 17) between the ventricles (A1, A2) and their arterial outlets (2, 3), carbon dioxide (41) and adrenaline (42) sensors as well as baroreceptors (39, 40) connected to an interactive electronic control device (45), detached to the outside and as an essential accessory comprises a cooling radiator (36) of the solenoid (35).

2. Device, according to claim 1, made of a rigid shell, intended to isolate the device from the rest of the body and act as a frame for internal anchoring of the associated devices, **characterised in that** the said rigid shell (1), ovoid in nature, comprises, first, on the outside, communication appendages for connecting to the natural adductors, two for the arteries (2, 3), equipped with elastic non-return valves (12, 13), and at least two for the veins (4, 5), and second, inside the said shell, a combination of intersecting partition walls, first (6), which insulates the transit and return B hydraulic spaces comprising two circular inlet ports (29, 30), equipped with non-return valves (31, 32) as well as two oblong outlet ports (43, 44), and the other subdividing partition (7), whose symmetrical plane is perpendicular to the isolating partition wall (6), such that, in contrast to the aforementioned appendages (2, 3), the said combination isolates two symmetrical, hydraulic transit and return pits (B1, B2), the said shell also comprises two four double-wall folded tubes, one (10) for the right half-heart and the other (11) for the left half-heart, such that these two tubes channel the venous blood flow from the transit hydraulic areas (B1, B2) via the oblong ports (43 and 44) of the partition wall (6), towards the oblong intake ports (27, 28) of the ventricular hydraulic spaces (A1, A2), the said tubes are fitted with lamellar valves (33, 34), and two others, one (8) for the right half-heart and the other (9), for the left half-heart, such that these tubes channel the blood flow from the outlet ports (14, 15) of the ventricular hydraulic spaces (A1, A2) to the arterial outlets, the said tubes (9, 10) are fitted with non-return lamellar valves (16, 17), the said shell, in its inner wall, also comprises the bilateral anchoring means, adapted to receive and fasten a supplementary cross structure.

3. Device, according to Claims 1 and 2, made of a cross structure that supplements the interior design with the shell (1), **characterised in that** the said structure comprises, from the anchor points of the inner wall of the shell (1), both the ventricular skirts (25, 26), integral with the holding frames (22, 23), the said frames are interconnected back-to-back through a skeletal linking structure (24), such that the said supplementary structure holds and shows the membranes (18, 19) and their discs, integrated, the permanent magnet (20, 21) and an interposed cooled solenoid (35).

4. Device, according to claims 1,2 and 3, hydraulic, with ventricular pump, **characterised in that** the ventricular membranes (18, 19) are made up of an elastomeric web preformed on the inside and the centre of which includes a permanent magnet disc (20, 21), embedded in elastomer F of the said membranes (18, 19), the said membranes are held at their periphery by the holding frames (22, 23), extended by the skirts (25, 26), anchored to the wall of the shell (1), such that this combination of means encloses the ventricular hydraulic space (A1, A2), the said membranes beat inside the said spaces (A1, A2) which comprise, open in the skirts (25, 26), the oblong intake ports (27, 28), served by the venous intake tubes (10, 11), as well as the oblong outlet arterial ports (14, 15) for the outlet arterial tubes (8, 9).

5. Device, according to claims 1, 3 and 4, solenoid, driving, in operation, generally animated, **characterised in that** the transverse structure (24) holds and shows the ventricular membranes (18, 19) and their included permanent magnets (20, 21) through frames (22, 23) such that the north pole of the one is facing the south pole of the other, and that the connecting structure (24) holds a cooled solenoid interposed between the two included permanent magnets (20, 21), such that its north pole repels the north pole inside one of the magnets while its south pole repels the south pole inside the other permanent magnet, the said device, through magnetic repulsion, induces the driven beating of the membranes, integrated with the included magnets, simultaneously in ventricular spaces (A1, A2) and in the pneumatic volumetric space D, such that during systole, the said beating compresses the ventricular hydraulic volumetric spaces (A1, A2), and simultaneously, depresses the pneumatic volumetric space D, and vice versa, during diastole.

6. Device, according to claims 1 and 5, driven, in operation, pneumatically animated driving the immersed atria, **characterised in that** after deducting the hydraulic volumetric spaces (A1, A2, B1, B2, C1, C2), and the tubes (8, 9, 10, 11) projecting into the shell (1), the remaining pneumatic volumetric space D, with variable volume, which separates the ventricular membranes (18, 19) and which houses the cooled solenoid (35) is, the said pneumatic space D, occupied by a gas depending on its density and its coefficient of elasticity in a state of slight vacuum during its relaxation phase at the end of diastole, such that the membranes (18, 19) simultaneously beat in the ventricular volumetric spaces (A1, A2), and in the pneumatic volumetric space D, in the opposite direction, and that due to this, the pneumatic atmosphere of said pneumatic volumetric space D is under variable vacuum, driven, due to the elasticity of the occupying gas, subjected to the action of said membranes.

7. Device according to claims 1 to 6, hydraulic, driven, in operation, with atrium C1, C2, **characterised in that** the said atria are made of elastic pipes, preformed, irregular, identical, enveloping and symmetrical, which join the connection appendages (4, 5) of the shell (1) to the hydraulic transit and return spaces (B1, B2), by ports (29, 30) opened in the isolating partition (6), the said pipes are immersed in the pneumatic atmosphere with variable vacuum of the pneumatic space D, such that changes in the pneumatic vacuum of space D act on the elasticity of the walls of the pipes (49, 50) which enclose the hydraulic atrium spaces (C1, C2), to induce variation in elasticity of said pipes, and their subsequent expansion under the action of internal pressure of the venous blood present in them and allow them to accept the said venous blood, in return, for temporary storage during systole.

8. Device according to claims 1 and 5, comprising a cooling device of the solenoid (35) in relation with the right half-heart, i.e., the half-heart serving the lungs, **characterised in that** it comprises a radiator (36), peripheral to the solenoid (35), the said radiator is traversed, in operation, by a small amount of blood diverted from the ventricular space A1 of the right half-heart, during systole, through a nozzle (37) equipped with a non-return valve (47), the said nozzle arranged in any hard part of the skirt (25) or in the intake tube (10), as shown in figure 1, wherein the nozzle is connected to the radiator (36) through an adduction tube fitted with a non-return valve (47), the said volume of taken blood is further channelled towards the arterial outlet (2) of the right half-heart via a tube which ends with a nozzle equipped with a non-return valve (48), placed as venturi in the said arterial outlet, downstream of the main non-return valve (12).

9. Device according to claims 1 and 2, comprising a non-return communication device, i.e. the circular valve that equips the appendages of the shell (2, 3) and the ports (29, 30) of the isolating partition (6) **characterised in that** the said non-return valve is made of a circular web (54), elastic, elastomeric, at the centre of which rises an integral mounting appendage, cylindrical, perpendicular to its plane, which is trapped in a gasket integral with a peripheral ring, concentric, by means of three hydrodynamic section branches, the said gasket, the connecting branches and the abovementioned ring form the mounting and presentation bracket (46) of the elastic web (54), such that said web rises and falls on the portion of the ring of its presentation bracket which acts as circular closing seat, the said bracket is installed in the ports (29, 30) of the partition (6), as well as in the arterial connection appendages (2, 3).

10. Device according to claims 1 and 2, comprising a non-return communication device, i.e., the lamellar valve, installed in the tubes (8, 9, 10, 11), **characterised in that** the said valve is made of two elastomer elastic blades (51), which on closing are supported on a rigid blade with hydrodynamic section having a leading edge and a trailing edge specific for avoiding turbulences, the said blade (52) is presented at the trailing edges of the said elastic blades by triangles (53), with which they form a trestle and on the edges of the said triangles the lateral trailing edges of the said elastic blades are supported to close the closing device during systole, in addition to circular valves.

11. Device according to claims 1, 4, 5 and 6, comprising a primary electronic initial switching device of the electrical pulse, **characterised in that** it comprises two baroreceptors (39, 40) installed on the hard parts of the left half-heart, in the internal side of the shell (1), one (40) within the ventricular space A2, and the other (39) in the hard parts of the atrium space C2 of the same left half-heart, such that coupled with the electronic control device (45) of the solenoid, this said device balances the immediate pressures and allows delivery of the necessary electric pulse to the solenoid (35) only for the starting of the systole.

12. Device according to claims 1, 4, 5, 6 and 11, comprising an electronic device for modulating and interrupting the electrical pulse of the solenoid (35), **characterised in that** the electronic control device (45), detached to the outside under the skin, is connected to the carbon dioxide (41) and adrenaline (42) sensors installed on the internal surface of the shell (1) in the ventricular space A1 of the right half-heart, such that, depending on the recorded rates of carbon dioxide and adrenaline, the said electronic control device modulates the intensity and duration of the electric pulse necessary for the moment's systole, and that the thus programmed duration, after reaching its end, closes the circuit opened by the above mentioned primary electronic switch, with which the device is also coupled to act in synchronised synergy.
